# EUROPEAN PATENT APPLICATION

(11) **EP 4 725 313 A1**
(43) Date of publication of application: **15.04.2026**
(21) Application number: 24746770.7
(22) Date of filing: 06.06.2024
(51) Int. Cl.: A01N 63/27, A01P 1/00, A01P 3/00, A01P 21/00, C12N 1/20

(54) **STRAIN OF PSEUDOMONAS AND ITS USES AS A BIOCONTROL AGENT AND AS A BIOSTIMULANT IN PLANTS**

(30) Priority: 06.06.2023 ES 202330452
(71) Applicant: Abiopep, S.L., Espinardo, 30100 Murcia (ES); Frutas y Hortalizas Del Sureste, S.L., 30700 Torre Pacheco (Murcia) (ES)
(72) Inventor: ARANDA REGULES, Miguel Ángel, 30100 Espinardo Murcia (ES); BERNAL VICENTE, Agustina, 30100 Espinardo Murcia (ES); SANCHEZ PUJANTE, Pedro Joaquín, 30100 Espinardo Murcia (ES); HERNANDO SAIZ, Yolanda, 30100 Espinardo Murcia (ES)
(74) Representative: Pons IP
(86) International application number: PCT/ES2024/070353
(87) International publication number: WO 2024/252050

(57) **Abstract**

The present invention relates to a new bacterial strain of the genus *Pseudomonas,* with deposit number CECT 30310, and its use as a biocontrol agent against a plant pathogen, preferably against *Olpidium virulentus* sp., vector of the viruses that cause lettuce big-vein disease and/or as a biostimulant for plant growth, as an agent inducing stress resistance in a plant.

## Description

The present invention belongs to the technical field of microbiology, particularly to agricultural microbiology. The present invention relates to a new strain of *Pseudomonas,* with deposit number CECT 30310, and its use as a pathogen biocontrol agent, preferably against *Olpidium virulentus* sp., vector of the viruses that cause lettuce big-vein disease and/or as a biostimulant of plant growth and as an agent inducing resistance to stress in a plant.

### BACKGROUND OF THE INVENTION

Intensive agriculture involves the application of significant quantities of phytosanitary products and chemical fertilisers, which often leads to the accumulation of heavy metals, and to the decrease in microbial diversity and nutrients available for the crop. Furthermore, the continued use of fertilisers poses a threat to the environment due to nitrate leaching, surface washing of phosphorus and nitrogen and eutrophication of aquatic ecosystems, with the increase in the concentration of nitrogen in the water that can be consumed (Vivian Z., Larsona J. A., Yinb, X., Savoyc H. J., McClureb A. M., Essingtonc M. E. and Boyera Ch. N. (2018). Profitability of enhanced efficiency urea fertilizers in no-tillage corn production. Agron. J. Abs. - Crop Econ. Prod. Manag. 110(4):1439-1446*).* The use of microorganisms as biostimulation or biocontrol agents is being researched to maintain agricultural productivity and preserve the environment. Therefore, integrated fertilisation management using microorganisms can improve nutrient uptake and fertiliser efficiency, reduce the quantities of these chemicals and the contribution of phosphorus or nitrogen (Satyaprakash M., Nikitha T. and Reddi E. U. B. (2017). Phosphorous and phosphate solubilising bacteria and their role in plant nutrition. Int. J. Curr. Microbiol. App. Sci, 6 (4): 2133-2144*).*

Biofertilisers are natural organic fertilisers that contribute to improve the availability of the necessary nutrients to plants and enhance soil quality by creating a natural microbiological environment, which reduced the population of phytopathogenic microorganisms. A large number of bacteria are known that stand out for their potential as biofertilisers (Prabakaran Elavarasi, Muthuraman Yuvaraj and Pandurangan Gayathri. (2020). Application of Bacteria as a Prominent Source of Biofertilizers. doi: 10.5772/intechopen.89825), including the genus *Pseudomonas.* The positive effects that these bacteria have on plants lie in the production and segregation of growth regulators such as auxins, gibberellins and cytokinins, which improve seed germination, mineral nutrition, root development and water absorption, among others.

Biostimulants act on the physiology of the plant, improve crop vigour, yield and quality of harvest, the availability and absorption of nutrients, and increase tolerance to abiotic stresses. Biostimulants and biofertilisers can have positive effects in mitigating diseases caused by phytopathogenic microorganisms. As they contribute to the modification of the plant metabolism, making them more tolerant to the symptoms of the disease, promoting their growth by masking the symptoms and/or occupying ecological niches that could be occupied by pathogenic microorganisms (Bhattacharyya P. & Jha D. (2012). Plant growth-promoting rhizobacteria (PGPR): emergence in agriculture. World J. Microbiol. Biotechnol., 28(4), 1327-1350).

Control of soil-borne plant diseases is difficult because, among other reasons, of the limitation of active materials available for controlling soil pathogens. This is the case of Lettuce Big-Vein Disease (LBVD), which is responsible for significant quality and yield losses in lettuce crops around the world. Affected plants show a lightening of the veins, with a characteristic appearance of thickened veins, ruffling of the leaves, reduction in bud size and significant decrease in the quality of the edible product (Maccarone L. D., et al 2013. Relationships between the pathogen Olpidium virulentus and viruses associated with lettuce big-vein disease. Plant Dis. 97, 700-707*).* LBVD can cause production losses of up to 70% in certain regions, and incidences close to 100% during winter and early spring are common in some geographic areas.

Mirafiori lettuce big-vein virus (MiLBVV; genus *Ophiovirus* spp.) together with Lettuce big-vein associated virus (LBVaV; genus *Varicosavirus* spp.) cause the disease (Sasaya, T., Fujii, H., Ishikawa, K. and Koganezawa, H. (2008). Further evidence of Mirafiori lettuce big-vein virus but not of Lettuce big-vein associated virus with big-vein disease in lettuce. Phytopathology 98, 464-468). Both viruses are transmitted by zoospores of the chytridiomycota fungus, *Olpidium virulentus,* obligate root parasite (Hartwright, L. M., Hunter, P. J. and Walsh, J. A. (2010). A comparison of Olpidium isolates from a range of host plants using internal transcribed spacer sequence analysis and host range studies. Fungal Biol. 114, 26-33; Maccarone, L. D., Barbetti M. J., Sivasithamparam K. and Jones R. A. C. (2010). Molecular genetic characterization of Olpidium virulentus isolates associated with big-vein diseased lettuce plants. Plant Dis. 94, 563-569). Resistance spores can remain dormant in the soil for a long time, and both MiLBVV and LBVaV can survive up to 20 years therein. To that end, the inoculum of both viruses can last for years, making control of LBVD a challenge once the fungus is established in the field. There are no effective control strategies for O. *virulentus* as it infects a wide range of weed species that act as reservoirs (Navarro J. A., Botella F., Marhuenda A., Sastre P., Sánchez-Pina M. A., and Pallas V. (2005). Identification and partial characterisation of Lettuce big-vein associated virus and Mirafiori lettuce big-vein virus in common weeds found amongst Spanish lettuce crops and their role in lettuce big-vein disease transmission. Eur. J. Plant Pathol. 113, 25-34).

There are currently few biostimulants or biofertilisers on the market formulated based on microorganisms, and none are specifically described as a biofertiliser or biostimulant for lettuce cultivation, much less as a LBVD control product.

Due to the foregoing, and given the great economic problem that this disease represents, ecological alternatives must be sought that prevent or reduce its incidence in the crop, based on the use of microorganisms that are applicable both in the seedbed and in the field and capable of surviving in the substrate in which they are inoculated.

### DESCRIPTION OF THE INVENTION

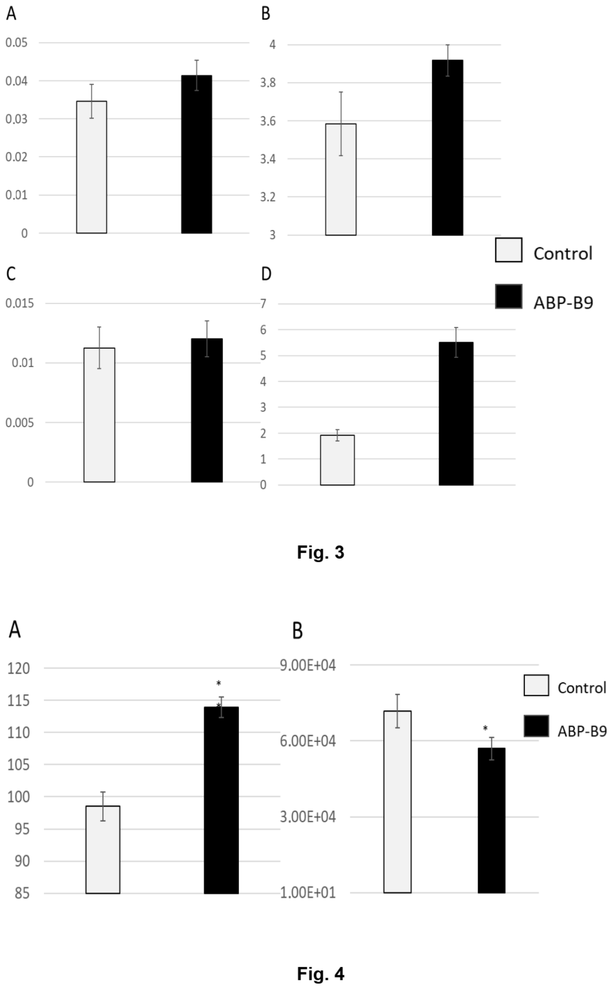

The present invention relates to a new strain of *Pseudomonas seleniipraecipitans,* the strain ABP-B9 with deposit number CECT 30310, with a high biostimulant capacity on plants, particularly lettuce, spinach, tomato, celery and melon, which generates an increase in the development of the root system, by producing a greater number of secondary rootlets, which can improve nutrient absorption (Figure 3, 5, 9, 10 and 13), both in the seedbed and in the field, and leads to an increase in plant development.

Furthermore, this strain is capable of minimising the symptoms of lettuce big-vein disease (LBVD) and reduce the number of spores produced *in vitro* by the fungus *Olpidium virulentus,* LBVD vector. Therefore, it has a biocontrol effect on the microorganisms responsible for the disease (Figure 4, 12 and 13), providing an ecologically sustainable solution against LBVD, thus said strain or a composition comprising same, could be applied both in conventional and in organic cultivation.

Therefore, a first aspect of the invention is a strain of *Pseudomonas seleniipraecipitans* deposited in the Spanish Type Culture Collection (CECT) with deposit number CECT 30310, hereinafter "strain of the invention".

The strain of the invention was isolated from the root system of lettuce plants. The strain was deposited on 31 March 2021 under the Budapest Treaty in the Spanish Type Culture Collection as the International Deposit Authority (main office at Edificio 3 CUE, Parc Científic Universitat de Valencia, C/ Catedrático Agustín Escardino, 9, 46980 Paterna (Valencia) SPAIN). The assigned deposit number was CECT 30310. The depositor of the strain was ABIOPEP S.L.

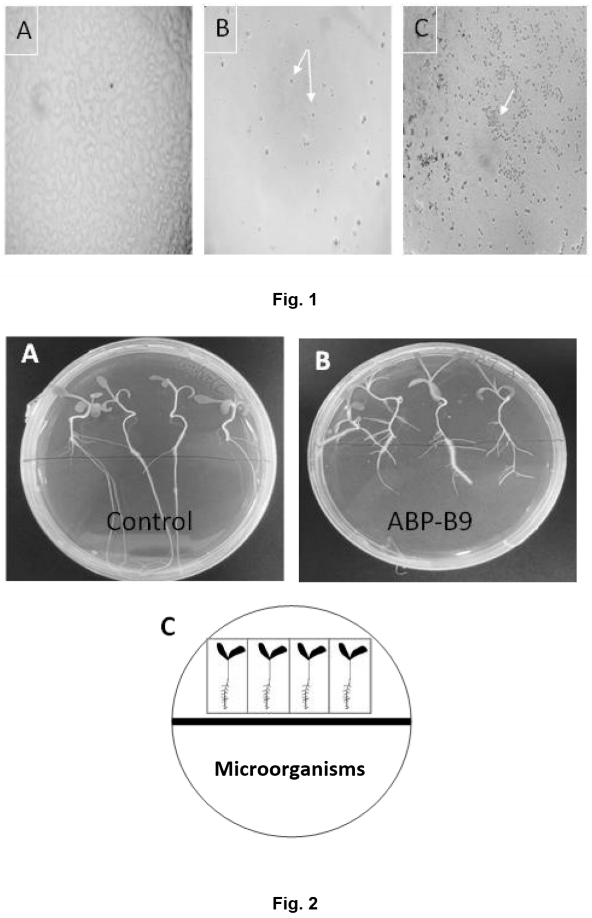

The strain of the invention is a bacterium that belongs to the genus *Pseudomonas* spp., particularly of the species *Pseudomonas seleniipraecipitans* sp., yellow-pigmented Gram-negative bacteria that reduce selenite to elemental red selenium, capable of promoting or stimulating plant growth, as well as minimising the symptoms of LBVD and reducing the number of spores produced *in vitro* by the fungus *Olpidium virulentus* (Figure 1).

The present invention also contemplates the microorganisms or bacteria that derive from the strain of the invention and that retain the ability to increase or promote the growth of plants and minimise the symptoms of diseases caused by pathogens, particularly the viral aetiology disease LBVD. Examples of strains or microorganisms derived from the strain of the invention may be mutants that present variations in their genome with respect to the genome of the strain of the invention.

Therefore, in another aspect, the present invention also relates to a strain derived from the strain of the invention. In the present document, the terms "mutant" or "derivative", used interchangeably, refer to any microorganism resulting from a mutation or modification in the DNA of an organism that results in a character (phenotype) that is not found in the wild type (wt) and that maintains the characteristics of the wild type related to the ability to biostimulate plant growth and biocontrol against pathogens.

The strain derived from the strain of the invention can be produced naturally, or intentionally by mutagenesis methods known in the state of the art such as, for example, mutagenic or stress-causing agents, or by genetic engineering aimed at modifying specific genes. Therefore, genetically modified mutants derived from the strain of the invention are also part of the present invention.

In another particular embodiment, the strain of the invention may be in the form of viable cells. The term "viable cells" used in the present invention refers to living or viable cells that when supplemented or incubated in an appropriate medium, are capable of dividing and reproducing.

Another aspect of the present invention relates to a biologically pure culture of the strain of the invention, hereinafter the "biologically pure culture of the invention". In this document, "biologically pure culture" is understood as the culture in which the microorganism or strain of the invention is found in a proportion equal to or greater than 99% with respect to the rest of the possible microorganisms present in the culture, and that may contain metabolites derived from the strain of the invention.

As understood by a person skilled in the art, the strain of the invention may be included within a biostimulant composition and/or a biocontrol composition against pathogens. Therefore, another aspect of the present invention relates to a composition comprising the strain of the invention and/or a bacterial culture medium, hereinafter "composition of the invention".

In another particular embodiment of the present invention, the composition of the invention comprises the strain of the invention in the form of a solution and more preferably at a concentration between 1·10³ and 1·10¹³ Colony Forming Units (CFU) per unit volume, particularly CFU/ml of solution. In another more particular embodiment of the invention, the concentration of the strain of the invention is between 10⁶ and 10¹¹ CFU/ml, preferably it is between 10⁷ and 10¹⁰ CFU/ml, both limits included. Preferably, the concentration of the strain of the invention is 10⁶, 10⁷, 10⁸, 10⁹ or 10¹⁰ CFU/ml.

As understood by a person skilled in the art, the strain of the invention can be comprised in any solution of the state of the art that preserves and maintains the viability of the cell culture and that allows the development of the strain of the invention. Examples of solutions of the strain of the invention, include but are not limited to, water, salts such as KCI or NaCl and/or nutrients (carbon and/or nitrogen sources).

As understood by a person skilled in the art, the composition of the invention further comprises at least one excipient or co-formulant that facilitates and helps increase plant development and growth and/or biocontrol against pathogens.

Therefore, in another particular embodiment of the invention, the composition of the invention further comprises at least one excipient or co-formulant. In this document the term "excipient" or "co-formulant" refers to a composition, solution, mixture, element or product, which is not the strain of the invention, and which is used in agriculture or gardening to facilitate, promote and assist plant growth, improve the quality or fertility of the soil, increase the viability and stability of the strain of the invention, improve the quality of the composition of the invention and its uses as a biostimulant and/or biocontrol agent against pathogens.

As understood by a person skilled in the art, the composition of the invention may further comprise at least one adjuvant. In the present document, the term "adjuvant" refers to a substance, compound or product that is incorporated into the composition of the invention to improve its behaviour and functionality, as well as, making the mixture of products compatible and stabilising the same. Therefore, in another particular embodiment of the present invention, the composition of the invention further comprises an adjuvant, preferably wherein this adjuvant is selected without limitation, from the list consisting of: dispersing agents, pH regulators, thickeners, re-suspension agents, preservatives, minerals, salts, acids, bases, stabilisers, and any combination thereof.

In a particular embodiment of the present invention, the strain or composition of the invention is in a solid state (for example, lyophilised), liquid, gelled or colloidal, or gaseous; preferably in liquid or solid state.

As described above, the strain of the invention enhances and increases the development and growth of plants and minimises the symptoms of LBVD, reducing the number of spores produced by the fungus O. *virulentus.* Therefore, another aspect of the invention is the use of the strain or composition of the invention as a biostimulant for the growth of a plant, as an agent inducing stress resistance in a plant and/or as a biocontrol agent against a plant pathogen, to increase, stimulate and enhance plant growth and/or minimise LBVD symptoms, hereinafter "use of the invention".

The term "biostimulant", used in the present invention, refers to an agriculturally acceptable product comprising live microorganisms, specifically the strain of the present invention, that when applied to plants, isolated plant material, rhizosphere or seeds, of crops; promotes, improves, and increases plant development or growth, in other words, promotes the growth or development of the root system of plants, the elongation of the stems, flowering and/or development of fruits and seeds, helping to improve the yield and quality of agricultural crops, improves nutrient uptake, nutritional efficiency and stress tolerance and also protects against pathogens.

As understood by a person skilled in the art, any species of plant can be used to stimulate its growth. Examples of plants include, but are not limited to, plants of agronomic or crop interest (for example, plants of interest in human or animal nutrition or plants of forestry interest) and plants of ornamental interest. Examples of plants of agronomic interest include, but are not limited to, oats, barley, corn, wheat, olive, grapevine, almond, soybean, beet, potato, carrot, garlic, rice, onion, tomato, sunflower, pepper, melon, lettuce, spinach, celery, rocket, broccoli, strawberry, bean, orange, apple, peach, lemon and pear, preferably lettuce (*Lactuca* spp.), spinach (*Spinacea* spp.), tomato (*Solanum* spp.), celery (*Parsley* spp.), or melon (*Cucumis* spp.).

In another particular embodiment of the use of the invention, the plant belongs to the genus *Lactuca* spp, *Cucumis* spp, *Spinacea* spp., *Solanum* spp., or *Apium* spp.

In another more particular embodiment of the use of the invention, the plant belongs to the species *Lactuca sativa sp., Cucumis melo sp., Spinacea oleracea sp., Solanum lycopersicum sp., or Apium graveolens sp.*

In this document the term "agent inducing stress resistance in a plant" refers to a substance, an agent, natural medium, living being or microorganism, of natural origin, that controls, eliminates, reduces, stabilises biotic, abiotic or environmental and phytotechnical stress conditions, in which the plant does not perform its physiological functions normally, which stops its growth and development, thereby limiting crop productivity.

In this document the term "biocontrol agent or biological control against pathogens" refers to a substance, an agent, natural medium, living being or microorganism, of natural origin, that controls, eliminates, reduces, stabilises pests or diseases caused by pathogens in plants, crops or agricultural crops, as well as the population of phytopathogens. Examples of phytopathogens include, but are not limited to, nematodes, bacteria, viruses, fungi, protozoans, molluscs, and insects.

In another particular embodiment of the invention, the strain or composition of the invention is used as a biocontrol agent against a pathogen, where the pathogen is a fungus or a virus.

Particularly, the strain or composition of the invention minimises the symptoms of LBVD, reducing the number of spores produced by the fungus *Olpidium virulentus.* Mirafiori lettuce big-vein virus (MiLBVV; genus *Ophiovirus* spp.) and Lettuce big-vein associated virus, (LBVaV; genus *Varicosavirus* spp.) cause LBVD. Both viruses are transmitted by zoospores of the chytridiomycota fungus, *O. virulentus,* which is an obligate root parasite.

Therefore, in another more particular embodiment of the invention, the strain or composition of the invention is used as a biocontrol agent against a fungus, where the fungus belongs to the genus *Olpidium* spp, preferably belongs to the species *Olpidium virulentus* sp.

In another more particular embodiment of the invention, the strain or composition of the invention is used as a biocontrol agent against at least one virus, where the virus belongs to the genus *Ophiovirus* spp. and/or *Varicosavirus* spp.

In another even more particular embodiment of the invention, the strain or composition of the invention is used as a biocontrol agent against a virus, where the virus that belongs to the genus *Ophiovirus* spp. is MiLBVV and/or the virus that belongs to the genus *Varicosavirus* spp. is the LBVaV virus.

As understood by a person skilled in the art, the use of the strain or composition of the invention may present biostimulant effects, such as those described above, or biocontrol against pathogens, separately or in combination (dual biostimulant/biocontrol agent activity).

As described above, the strain of the invention can be used in a method to increase the development and growth of plants and/or minimise the symptoms of disease caused by pathogens.

Therefore, another aspect of the present invention is a method of increasing or promoting the growth of a plant, to increase the stress resistance of a plant and/or for the biocontrol of pathogens in a plant, hereinafter the "method of the invention", that comprises:
(i) contacting a plant or plant material isolated from said plant with the strain of *Pseudomonas seleniipraecipitans* CECT 30310 or with the composition of the invention.

The strain of the invention and the composition of the invention have already been described in previous paragraphs of this document and apply equally to this inventive aspect, as well as all their particular embodiments. Likewise, the expressions "increase the growth of a plant", "agent inducing stress resistance in a plant" or "biocontrol of pathogens" have been described previously herein and equally apply to this inventive aspect.

In another particular embodiment of step (i) of the method of the invention, the plant belongs to the genus *Lactuca* spp, *Cucumis* spp, *Spinacea* spp., *Solanum* spp., *Apium* spp., preferably the plant belongs to the species *Lactuca sativa sp., Cucumis melo sp., Spinacea oleracea sp., Solanum lycopersicum sp., or Apium graveolens sp.*

As described above, the strain or composition of the invention is used in the method of the invention for biocontrol against a pathogen. Therefore, in another particular embodiment of the method of the invention, the pathogen is a fungus or a virus.

In another more particular embodiment of the method of the invention, the fungus belongs to the genus *Olpidium* spp., preferably to the species *Olpidium virulentus* sp.

In another more particular embodiment of the method of the invention, the virus belongs to the genus *Ophiovirus* spp. and/or *Varicosavirus* spp.

In another even more particular embodiment of the method of the invention, the virus that belongs to the genus *Ophiovirus* spp. is MiLBVV and/or the virus that belongs to the genus *Varicosavirus* spp. is LBVaV.

The strain or composition of the invention can be inoculated or applied to plants, including all stages of development, or to a plant material isolated from said plant. The term "plant material isolated from plants", as it is used herein, refers to isolated plant cells, or isolated parts of a plant, as tissues and organs of a plant, which can lead to the development of a plant. Examples of plant material isolated from the plant include, but are not limited to, seeds, leaves, stems, hypocotyls, cotyledons, pollen grains, roots, root tips, anthers, ovules, flowers, seedlings, embryos and capsules, preferably the plant material isolated from the plant is a seed.

In another particular embodiment of the method of the invention, step (i) is carried out by applying the strain or the composition in liquid form, solid form (for example, lyophilised) or hydroponically, preferably in liquid form.

In another particular embodiment of the method of the invention, the strain or composition of the invention is administered at least once, preferably between one and five times, more preferably between one and three times.

In step (i), the method of the invention comprises contacting a plant or plant material isolated from said plant with the strain or composition of the invention by any known technique, such as, for example, but not limited to, through hydroponics, through a solution applied to the soil, by means of the application of the strain or composition by spraying, infiltration, sprinkling, coating, fumigation or impregnation of any part of the plant, seeds or plant material isolated from said plant or in irrigation water.

Subsequent to contact between a plant or plant material isolated from a plant and the strain or composition of the invention, said plant or plant material is developed by practices known in the state of the art. The term "developing the plant" refers to growing a plant or plant material isolated from a plant in a particular environment and conditions (growing medium, temperature, time, light and/or dark conditions) that allow the increase in the vegetative development and growth of said plant and/or biocontrol against pathogens.

For example, the development of the plant can be carried out in an agricultural medium or agriculturally acceptable medium, referring to an element, compound, product or solution, that is natural or synthetic, mineral or organic, that in pure form or in a mixture, allows the anchoring of the plant's root system both in soil and in hydroponic cultivation, therefore, supporting and holding for the plant or seeds, as well as facilitating the interaction in plants or agricultural crops with the strain or composition of the invention.

Therefore, in another particular embodiment of the invention, the agricultural medium of the method of the invention is selected from the list consisting of: organic substrate, inorganic substrate, water, saline solution or any combination thereof.

As is understood by a person skilled in the art, any soil, agriculturally acceptable organic or inorganic substrate is valid to be used in the present invention. Examples of agriculturally acceptable organic substrates include, but are not limited to, clay, plant waste, cork powder, cellulose, peat, coconut fibre or compost.

### DESCRIPTION OF THE FIGURES

**Figure 1****.** Optical microscopy images of the ABP-B9 bacterial strain growth and spores of *Olpidium virulentus* on agar-water plates (100X resolution). (A) Growth of ABP-B9. (B) Dual culture of ABP-B9 and O. *Virulentus.* (C) Growth of O. *virulentus.* Resistance spores of O. *virulentus* are indicated with arrows.
**Figure 2****.** Development of lettuce seedlings (Fernandola variety) on agar-water plates. (A) Plants inoculated with water (control), (B) with the ABP-B9 bacteria and (C) diagram of the plate layout.
**Figure 3****.** Development of the aerial part and root system of cultivated lettuce seedlings *in vitro* (agar-water plates) at 20 days post-germination with control treatment (water) and ABP-B9. (A) aerial part weight, (B) number of leaves, (C) root weight and (D) number of secondary rootlets.
**Figure 4****.** Effect on the vegetative development of lettuce plants (var. Fernandola) grown in a greenhouse (control treatment and with ABP-B9) of (A) average weight (g) of the aerial part and (B) number of molecules of O. *virulentus* present in its root system (relative quantification by RT-qPCR). The asterisk indicates statistical significant differences between the treatments, One-Way Anova test (p < 0.05).

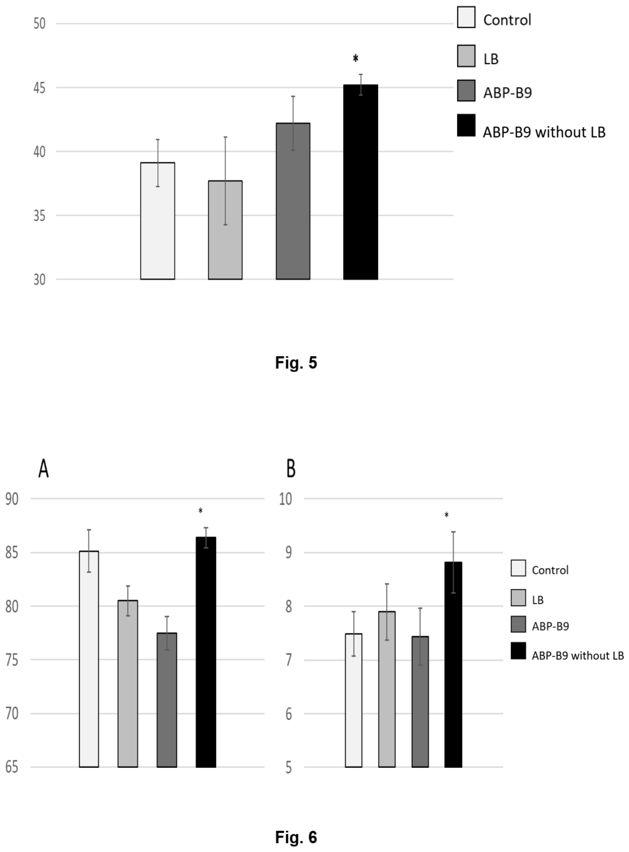

**Figure 5****.** Average fresh weight (g) of lettuce plants (Amenas variety) grown in a greenhouse with treatments: control (water); Luria-Bertani (LB) culture medium; ABP-B9 with LB medium (ABP-B9) and ABP-B9 without LB medium (ABP-B9 without LB). The asterisk indicates statistical significant differences between the treatments, One-Way Anova test (p < 0.05).
**Figure 6****.** Mean of the (A) relative water content (RWC) and (B) of the leaf mass per area (LMA) in lettuce plants (Amenas variety) grown in a greenhouse with treatments: control (water); Luria-Bertani (LB) culture medium; ABP-B9 with LB medium (ABP-B9) and ABP-B9 without LB medium (ABP-B9 without LB). Asterisks indicate statistical significant differences between treatments, One-Way Anova test (p < 0.05).

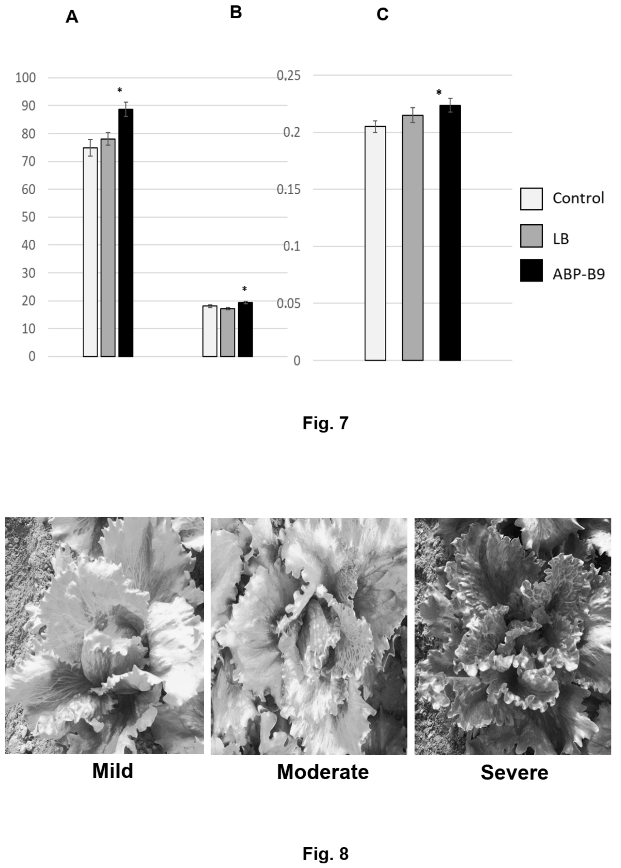

**Figure 7****.** Mean of (A) nitrogen balance index (NBI), (B) chlorophylls and (C) polyphenols (flavonoids), of lettuce plants (Amenas variety) grown in a greenhouse with control treatment (water), LB medium and ABP-B9. Asterisks indicate statistical significant differences between treatments, One-Way Anova test (p < 0.05).
**Figure 8****.** Scale for the evaluation of symptoms of lettuce big-vein disease (LBVD) used to evaluate plants subjected to different treatments.

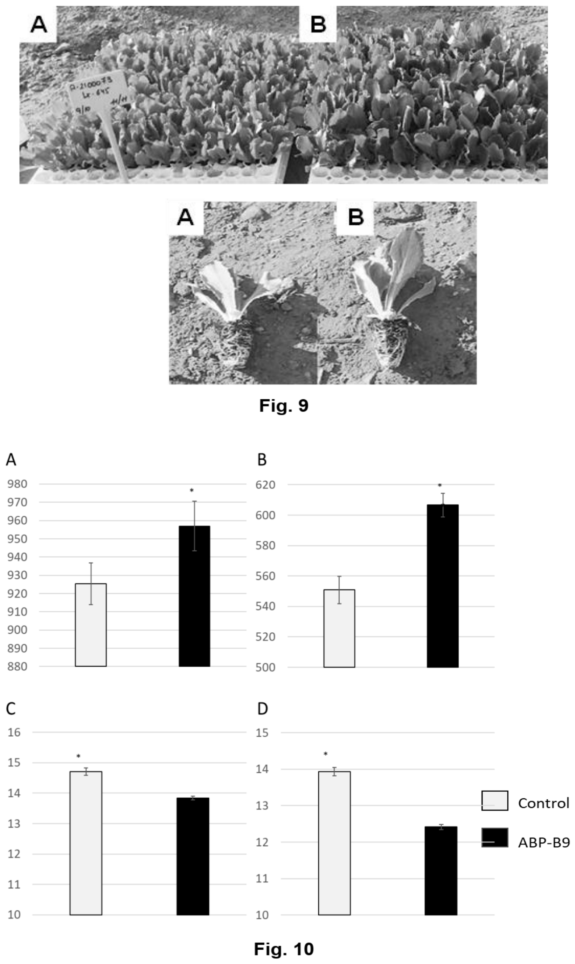

**Figure 9****.** Development of lettuce plants (var LI645) 5 days after inoculation in the seedbed. (A) Control treatment (water), (B) Treatment with ABP-B9.
**Figure 10****.** Mean (A) plant weight (g), (B) bud weight (g), (C) radial diameter (cm) and (D) polar diameter (cm), of lettuce plants (var. LI645) grown in the field, commercial crop with control treatment (water) and ABP-B9. Asterisks indicate significant differences, One-Way Anova test (p<0.05).

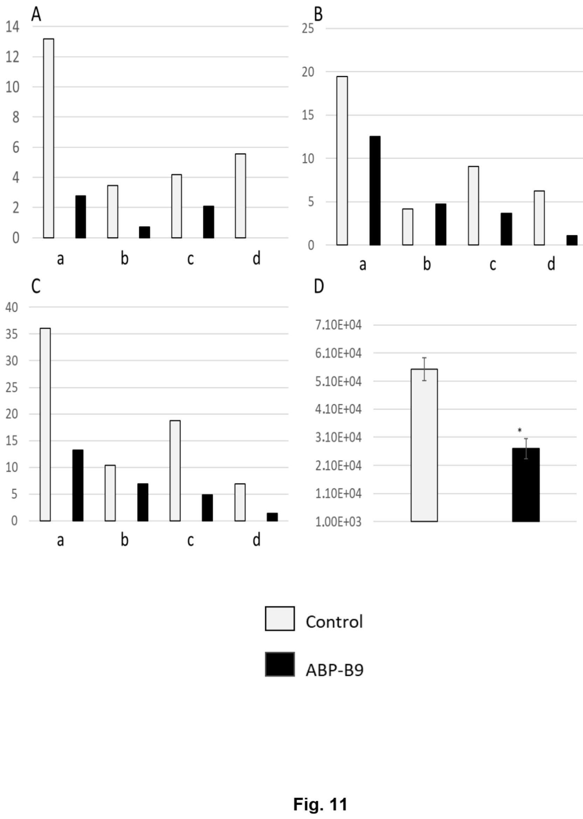

**Figure 11****.** Percentage of appearance of LBVD symptoms in lettuce plants (var. LI645) grown in the field, commercial cultivation, treated with control (water) and ABP-B9, at 51 (A), 59 (B) and 72 (C) days post transplant, in (a) total or depending on the severity of symptoms (b) mild, (c) moderate and (d) severe. (D) Number of molecules of *O*. *virulentus* present in its root system (relative measurement using RT-qPCR). Asterisks indicate significant differences, One-Way Anova test (p<0.05).

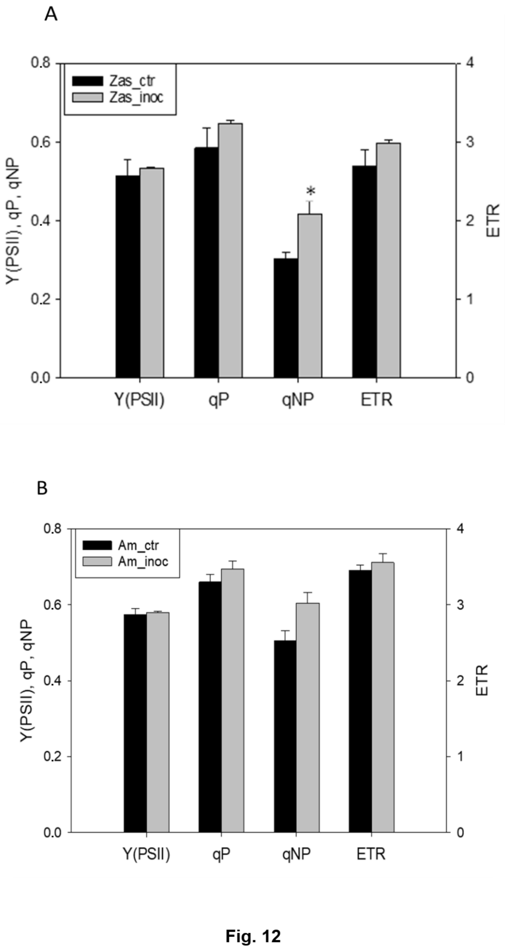

**Figure 12****.** Mean fluorescence parameters of lettuce plants grown in the field, commercial cultivation: (A) var. Zasmira (Zas) and (B) var. Amenas (Am): quantum yield of PSII [Y(PSII)], photochemical dissipation of fluorescence (qP), non-photochemical processes (qNP), and electron transport rate (ETR), with control treatment (ctr) (water) and ABP-B9 (inoc)). Asterisks indicate significant differences, One-Way Anova test (p<0.05).

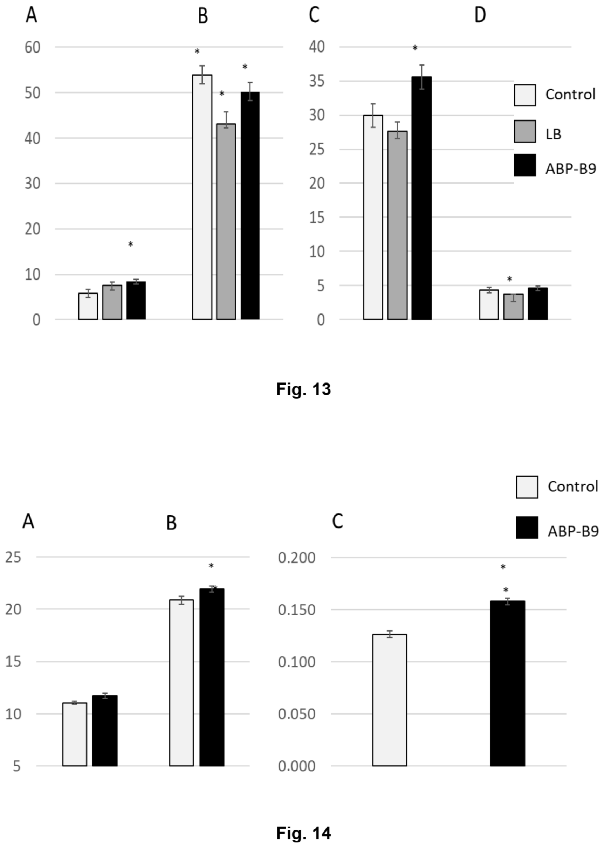

**Figure 13****.** Mean of (A) number of flowers, (B) stem length (cm), (C) fresh weight (g) and (D) dry weight (g) of melon plants (Charentais variety) grown in a greenhouse with treatments: control (water); Luria-Bertani (LB) culture medium and ABP-B9 with LB medium (ABP-B9). Asterisks indicate significant differences, One-Way Anova test (p < 0.05).
**Figure 14****.** Mean of (A) nitrogen balance index (NBI), (B) chlorophylls and (C) polyphenols (anthocyanins), of melon plants (Charentais variety) grown in a greenhouse, control treatment (water) and ABP-B9. Asterisks indicate significant differences, One-Way Anova test (p < 0.05).

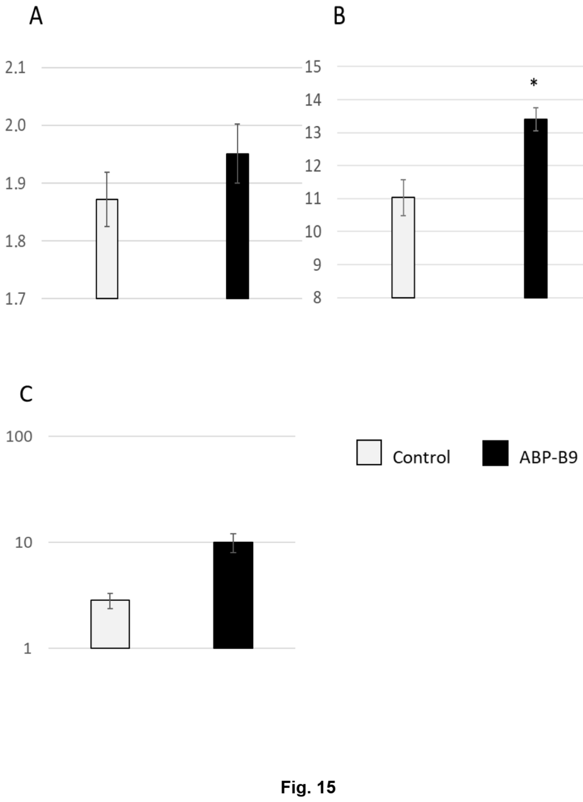

**Figure 15****.** Mean of (A) plant length (m), (B) dry weight of the root (%) and (C) ABP-B9 molecules in the root system (relative quantification by RT-qPCR, with Taq-Man probe), of melon plants (Loire variety) grown in the field, commercial cultivation, control treatment (water) and ABP-B9. The asterisk indicates significant differences, One-Way Anova test (p<0.05).

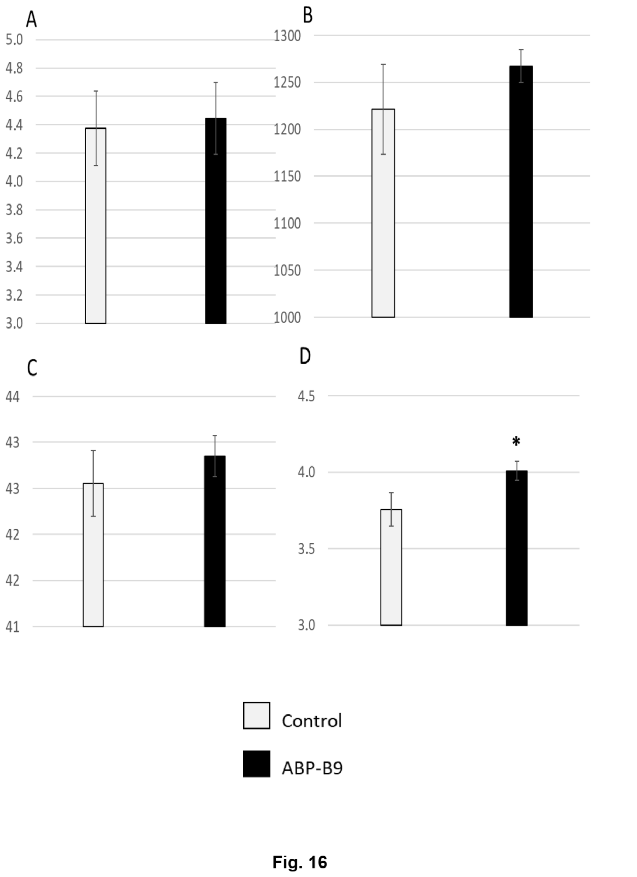

**Figure 16****.** Mean of (A) number, (B) Weight (g), (C) diameter (cm) and (D) pulp thickness (cm) of fruits of melon plants (Loire variety) grown in the field, commercial cultivation, control treatment (water) and ABP-B9. The asterisk indicates significant differences, One-Way Anova test (p<0.05).

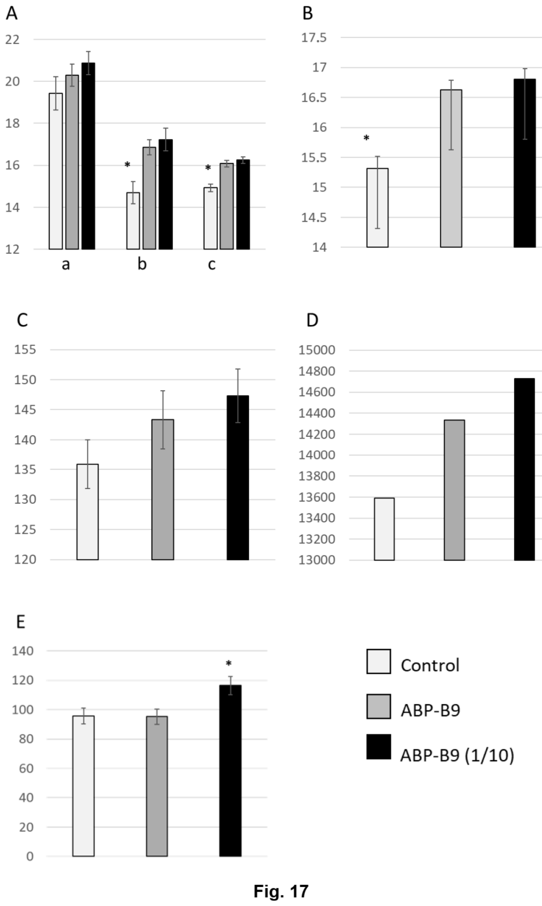

**Figure 17****.** Mean (A) of the weight of 10 ripe tomatoes (g) harvested at 53 (a), 60 (b) and 70 (d) days after transplant; (B) the weight of 30 ripe tomatoes harvested at the end of the trial; (C) mean number of tomatoes produced per plant; (D) estimate of the number of tomatoes produced by 100 plants and (E) average number of flowers produced per plant, control treatment (water), ABP-B9 and ABP-B9 (1/10). Asterisks indicate significant differences, One-Way Anova test (p<0.05).

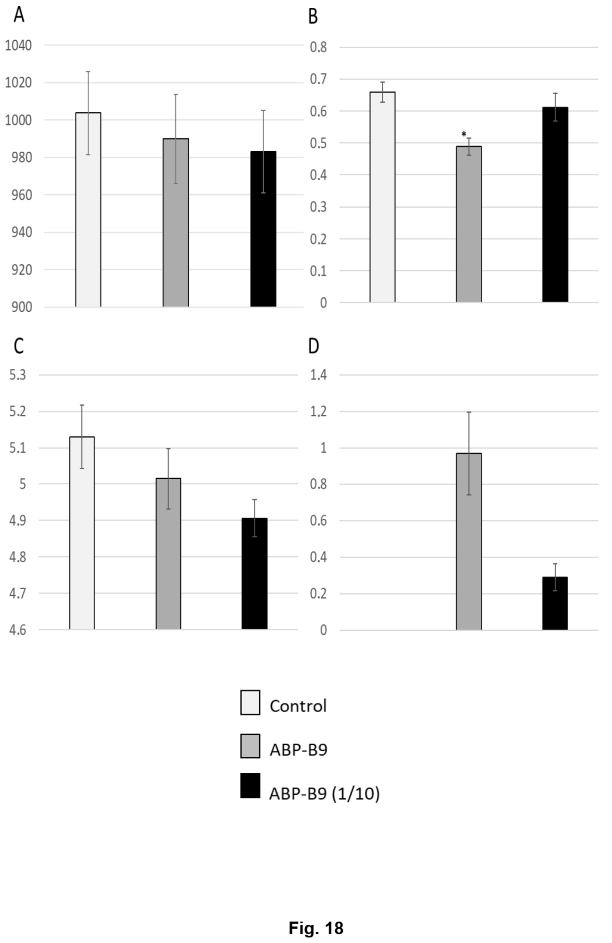

**Figure 18****.** Mean (A) of stomatal conductance (µmol m² s⁻¹) of 5 tomato plants, (B) acidity (citric acid equivalents) and (C) sugar content (° Brix) of 20 tomato plants and (D) mean number of ABP-B9 molecules in the root system (relative quantification by RT-qPCR with TaqMan probes), control treatment (water), ABP-B9 and ABP-B9 (1/10). The asterisk indicates significant differences, One-Way Anova test (p<0.05).

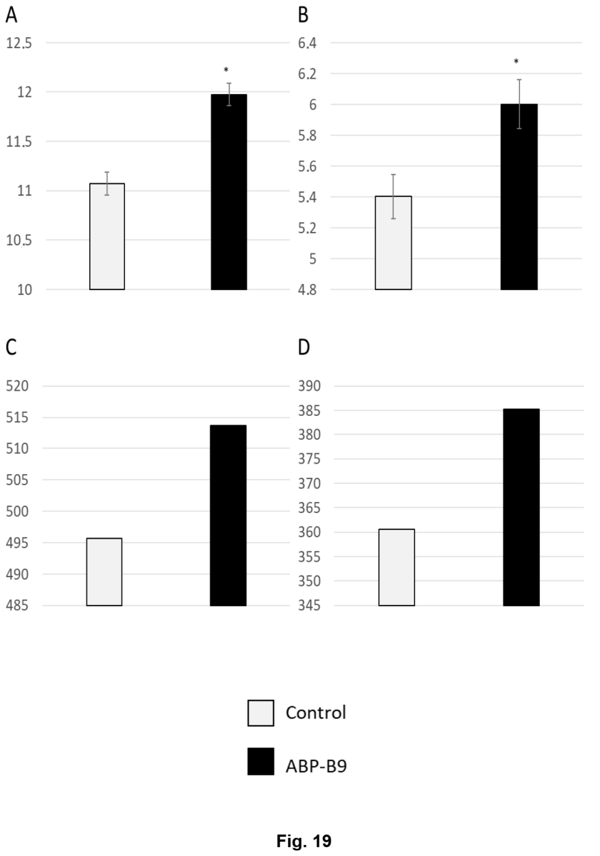

**Figure 19****.** Mean of (A) length (cm) and (B) weight (g) of the aerial part, (C) average weight (g) of 100 plants and (D) production estimate (Kg) of 100 m² of spinach, commercial cultivation in the field, control treatment (water) and ABP-B9. The asterisk indicates significant differences, One-Way Anova test (p<0.05).

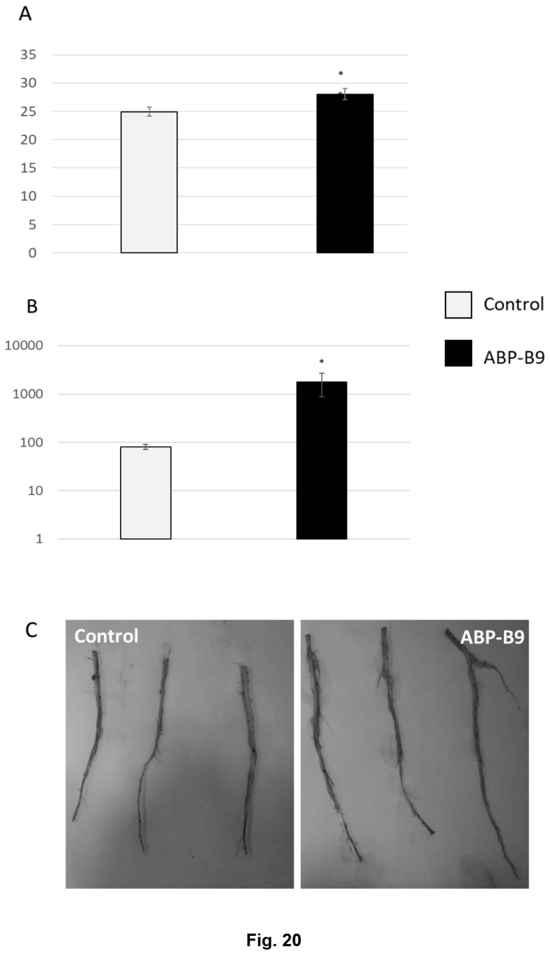

**Figure 20****.** Mean of (A) dry weight of the root (g); (B) of the number of ABP-B9 molecules (relative quantification by RT-qPCR, with Taq-Man probes) in the root system of spinach plants and (C) images of the root system of 3 spinach plants commercially grown in the field, control treatment (water) and ABP-B9. The asterisk indicates significant differences, One-Way Anova test (p<0.05).

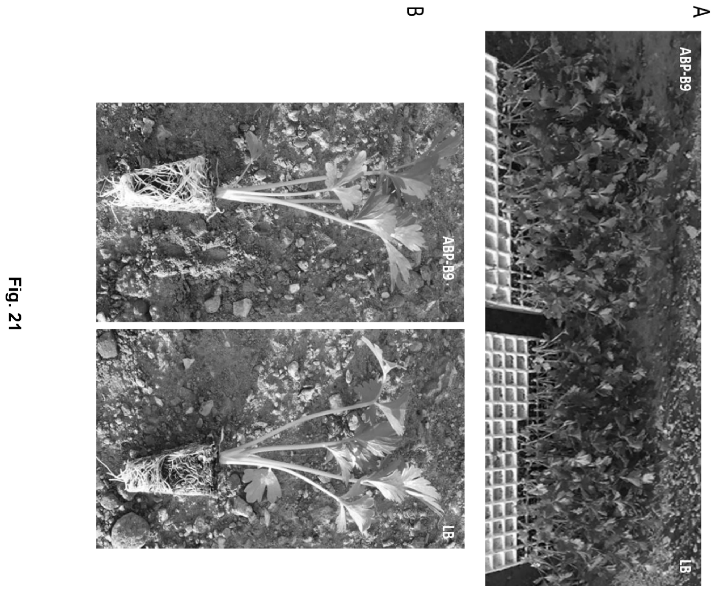

**Figure 21****.** Images of the development of celery seedlings 5 days after the application of the different treatments in the seedbed, (A) tray and (B) detail of seedlings, control treatments (water) and ABP-B9.

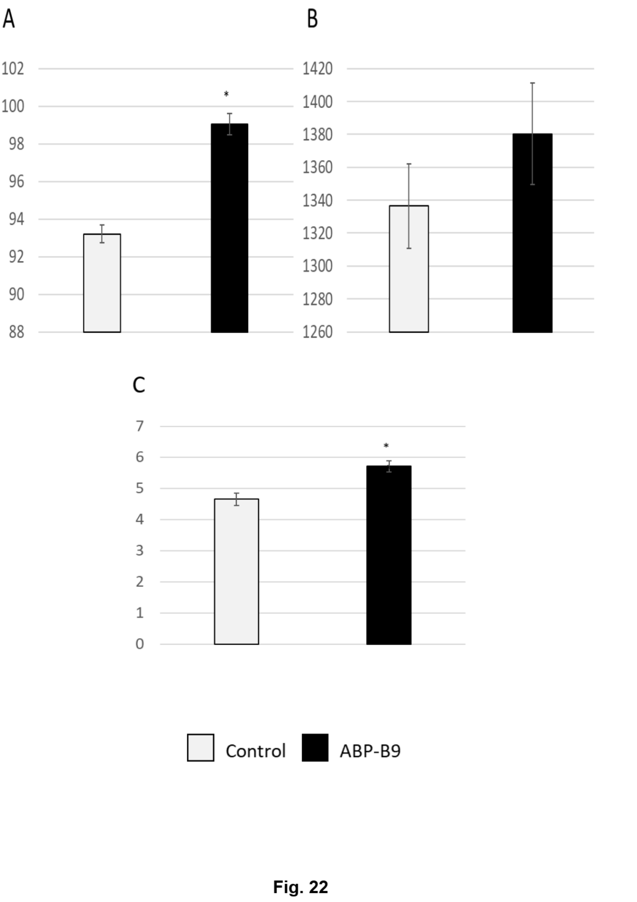

**Figure 22****.** Mean of (A) length (cm), (B) weight of the aerial part (g) and (C) dry weight of the root (g) of 100 celery plants grown in the field, commercial cultivation, control treatment (water) and ABP-B9. The asterisk indicates significant differences, One-Way Anova test (p<0.05).

### EXAMPLES

Next, the invention will be illustrated by means of trials carried out by the inventors which demonstrate the effectiveness of the product of the invention.

### 1. ISOLATION AND SELECTION OF ABP-B9

Microorganisms present in the root system of iceberg lettuce plants grown in cultivation plots in the municipality of Águilas in the Region of Murcia were isolated. To that end, the root system was sterilised with 10% sodium hypochlorite for 10 min, then, a homogenate was prepared with sterile distilled water, and serial dilutions were made, of which 100 µl were seeded on plates with LB Miller culture medium (Condalab). The plates were incubated at 28°C in the dark and the different microorganisms present were isolated.

The biocontrol capacity against *O*. *virulentus,* vector of the viruses responsible for LBVD, and biostimulant capacity was evaluated using lettuce seedlings by using dual plate culture, confronting each microorganism with a suspension of *O. virulentus* spores. After several weeks of incubation, the number of fungal spores was counted with an optical microscope. The bacteria that gave rise to the formation of the lowest number of spores was referred to as ABP-B9 (Figure 1).

Phylogenetic analysis of ABP-B9 was performed based on the housekeeping genes proposed for the genus *Pseudomonas,* 16S rRNA, gyrB, rpoB and rpoD. Phylogenetic trees using individual or concatenated alignments, were built using the software MEGA *7 (The Molecular Evolutionary Genetics Analysis Version 7.0*) (Kumar, S., Stecher, G. & Tamura K. (2016). MEGA7: Molecular Evolutionary Genetics Analysis Version 7.0 for Bigger Datasets. Molecular biology and evolution, 33(7), 1870-1874), showing that ABP-B9 had a high phylogenetic relationship with *Pseudomonas seleniipraecipitans* (Table 1).

**Table 1. Phylogenetic relationship with Pseudomonas seleniipraecipitans.**

| **Gen** | **Sequence with the highest percentage identity with ABP-B9** | **Accession Number** | **% identity** |
|---|---|---|---|
| **16S rRNA** | *Pseudomonas seleniipraecipitans strain IS1-14* | MF523618.1 | 100 |
| | *P. seleniipraecipitans_strain_CA5* | NR_116646.1 | 99.77 |
| | *Pseudomonas_seleniipraecipitans_strain_ PA100* | KT710798.1 | 99.37 |
| | *Pseudomonas_sp._PA35* | KT710797.1 | 99.23 |
| | *Pseudomonas_punonensis_strain_LMT03* | MT677939.1 | 99.07 |
| | *Pseudomonas_fulva_12-X_complete_genome* | CP002727.1_2395 22-241045 | 99.04 |
| **gyrB** | *P. seleniipraecipitans_strain_LMG_25475T* | HE800485.1 | 98.05 |
| | *Pseudomonas_punonensis_strain_LMT03* | JX435105.1 | 91.48 |
| | *Pseudomonas_seleniipraecipitans_strain_ PA100* | KT710837.1 | 91.17 |
| | *Pseudomonas_flavescens_LMG_18387T* | FN554183.1 | 90.73 |
| **rpoD** | *P. seleniipraecipitans_strain_LMG_25475T* | HE800485.1 | 98.02 |
| | *Pseudomonas_punonensis_strain_LMT03* | JX435105.1 | 86.61 |
| | *Pseudomonas_fulva_12-X_complete_genome* | NC_015556 | 86.22 |
| **rpoB** | *P. seleniipraecipitans_strain_LMG_25475T* | HE800485.1 | 99.12 |
| | *Pseudomonas_punonensis_strain_LMT03* | JX435105.1 | 95.51 |
| | *Pseudomonas_straminea-strain_LMG_21615T* | FN554758.1 | 93.55 |
| | *P. seleniipraecipitans_strain_CA5* | NR_116646.1 | 98.96 |
| **16s_gyrB_rpo D_rpoB** | *Pseudomonas_punonensis_strain_LMT03* | JX435105.1 | 93.80 |
| | *Pseudomonas_fulva_12-X_complete_genome* | CP002727.1_2395 22-241045 | 92.55 |
| | *Pseudomonas_straminea* | D84023.1 | 92.55 |

### 2. EVALUATION OF THE BIOCONTROL AND BIOSTIMULANT CAPACITY IN LETTUCE

These analyses were carried out in the laboratory (*in vitro*), using agar-water plates, and in a greenhouse using lettuce plants grown in pots (*in vivo*).

### 2.1. In vitro assay

Four lettuce seedlings were placed, pregerminated in sterile conditions, on agar-water plates previously inoculated with water (control treatment) or with the ABP-B9 bacteria that induces the formation of secondary rootlets (Figure 2). Five plates/treatment were incubated, at 25°C with a 16h:8h (light:dark) photoperiod and 60% humidity. After 2 weeks, the weight of the aerial part, of the root system, leaf and root count were determined. The seedlings treated with the ABP-B9 bacteria showed greater development, both of the aerial part (Fig. 3A), as well as the root system (Fig. 3C), a greater number of leaves (Fig. 3B) and secondary rootlets (Fig. 3D), 20 days post-germination compared to the control treatment (water).

### 2.2. In vivo assay

To corroborate the data of the *in vitro* assay, study the behaviour of the ABP-B9 bacteria and its effect on lettuce plants, trials were carried out *in vivo* in a greenhouse.

### 2.2.1. Evaluation of the effect of ABP-B9 on the development of lettuce plants and its biocontrol capacity against O. virulentus.

### • Evaluation of the biocontrol and biostimulant effect of ABP-B9 on lettuce plants

Lettuce seeds (Fernandola variety) were germinated in substrate (pro-line compost, coconut fibre and vermiculite, 2:1:0.1). At 30 days, were transplanted into pots that were inoculated with 5 ml of ABP-B9 (10⁸ CFU/ml), 5 replicates/treatment, in the presence or absence of *O. virulentus,* inoculated by adding 20 g of infected peat to each pot. Two treatments were carried out: control (inoculated with water) and inoculated with ABP-B9. The incorporation of *O. virulentus* produced a general decline in plant development, compared to plants developed in its absence. At 30 days post transplant (dpt), the weight of the aerial part of the plants and the population of *O. virulentus* in its root system were determined.

The results showed that ABP-B9 inoculation produced an increase in the development of the aerial part compared to the control (Figure 4A). Furthermore, plants inoculated with ABP-B9 had a lower population of *O. virulentus* than control plants (Figure 4B).

Therefore, the application of ABP-B9 results in an increase in plant development and has a biocontrol effect against *O*. *virulentus.*

### • Evaluation of the effect of ABP-B9 on the physiological state of lettuce plants

The evaluation of the development and physiological state of lettuce plants treated with ABP-B9 was carried out with the Amenas variety grown in a greenhouse in pots in the absence of fertilisers. Four treatments were carried out (6 plants/treatment): control (water), LB(LB medium), ABP-B9(10⁹ CFU/ml) with (ABP-B9) and without LB (ABP-B9 without LB), applying 5 ml of inoculum in all cases.

The analysis of the development of the plant and its physiological state was carried out at 30 dpt. The (i) weight of the aerial part (fresh weight), (ii) relative water content (RWC) of the leaf, (iii) leaf mass per area (LMA), (iv) chlorophylls, (v) polyphenols (Flavonoids) and (vi) nitrogen balance index (NBI) were determined using the Dualex Scientifc+TM optical meter (FORCE A; France).

The analysis of the results showed that the application of ABP-B9 produced an increase in the fresh weight of the plants (Figure 5), due to the action of the microorganism and not to the nutrients provided by the culture medium (LB). Furthermore, the microorganism does not negatively affect the development of the plants (Figure 6) and significantly increases their nitrogen status (Fig. 7A), chlorophyll levels (Fig. 7B) and flavonoids (Fig. 7C), which increases its photosynthetic capacity.

### 2.2.2. Evaluation of the effectiveness of ABP-B9 in lettuce cultivation under commercial production conditions

Field cultivation is a more complex environment for the development of the plant and the survival of the inoculated microorganisms. Therefore, a field trial was carried out to evaluate the effect of ABP-B9 on plants under commercial cultivation conditions and during the time of highest incidence of the disease (LBVD).

The assay was carried out in Águilas (Murcia, Spain) and consisted of two treatments (control, (inoculated with water) and ABP-B9), 6 replicates/treatment distributed in randomised blocks of 25/block, 150 plants (variety LI 645)/treatment. The treatments were separated by placing 20 untreated plants to avoid possible contamination. The seeds were germinated in a seedbed for transplanting to the field after 30 days. Five days before this transplant, 5 ml were inoculated of ABP-B9/seedling (10⁹ CFU/ml).

The development of the culture and the LBVD condition were visually monitored, and at the end, the 150 plants of each treatment and their separation were sampled. At 72 dpt weight of the aerial part, of the bud, radial and polar diameters and the amount of O. *virulentus* in the root system (30 plants/treatment) were determined. To evaluate the symptoms, a previously developed scale was followed (Figure 8).

Five days after inoculation, greater development was observed in the plants inoculated with ABP-B9 compared to the control (Figure 9), an increase that was still maintained 20 days after transplanting in the field. Plants inoculated with ABP-B9 showed a significant increase in development, with a greater fresh weight of the aerial part and the bud (Figure 10A and 10B). These plants showed a smaller radial and polar diameter of the bud than those that were treated with water (control), indicating that ABP-B9 induces greater compaction of the bud (Figure 10C and 10D).

The first symptoms of LBVD were observed at 40 dpt. From the beginning, the plants inoculated with ABP-B9 showed very little incidence of the disease and those that showed symptoms were mostly mild in nature (Figure 11A, 11B and 11C). This situation was maintained until the end of the trial (72 days). The analysis of the population of O. *virulentus* in the root system showed that inoculation with ABP-B9 significantly decreases the pathogen population (Figure 11D).

### Effect of ABP-B9 on photosynthetic efficiency

To find out if the plants suffered any type of stress, their physiological state was evaluated by determining the photosynthetic performance of photosystem II (PSII) (10 plants/treatment), using a modulated fluorescence fluorometer. This analysis was carried out on two commercial varieties of iceberg lettuce, Amenas and Zasmira, treated with the microorganism ABP-B9. The treatment showed an increase in the photochemical process or photochemical quenching, measured as Y(PSII) and qP, which was more pronounced in the Zasmira variety (Figure 12A and 12B). This indicates greater photosynthetic efficiency in plants treated with ABP-B9 than in untreated plants. The differences observed in the electron transport rate (ETR) between the treatment and the control, were not significant, probably due to the low number of samples analysed (Figure 12A and B). Non-photochemical quenching (related to the dissipation of excess energy in the form of heat and the response to stress), measured as NPQ or qNP, increased significantly compared to the control in the 2 varieties (P<0.05) (Figure 12A and 12B), which indicates that treatment with ABP-B9 increases the capacity of lettuce plants to respond to both biotic and abiotic stress situations.

### 3. EVALUATION OF THE BIOCONTROL AND BIOSTIMULANT CAPACITY IN MELON

### 3.1. Evaluation of the effect of ABP-B9 on the development and physiological state of Charentais melon plants

The evaluation of the effect of ABP-B9 on melon was carried out with the Charentais variety in a greenhouse, 6 replicates/treatment, in the absence of fertiliser. The seeds were disinfected, pregerminated and transplanted into pots with substrate. When the plants developed the first true leaf, they were inoculated with 5 ml of water (control), of LB or ABP-B9 (10⁹ CFU/ml). The assay ended at 30 dpt, when the following was determined: (i) weight of the aerial part (fresh and dry), stem length and number of leaves and flowers of each plant, (ii) relative water content (RWC) of the leaf, (iii) leaf mass per area (LMA), (iv) electrolyte loss (EL) and (v) chlorophyll determination, polyphenols (flavonols and anthocyanins) and the nitrogen balance index (chlorophyll-flavonol ratio), using the Dualex Scientifc+TM optical meter (FORCE A; France).

The results obtained together with the visual analysis of the development of the root system allowed us to conclude that the application of ABP-B9 to melon plants had no negative effects on the development of the crop,and led to an increase in the fresh weight and number of inflorescences of the treated plants (Fig. 13). Furthermore, this application does not pose a stress for the plant since the RWC, LMA and electrolyte loss parameters remained at the same level as those recorded for control plants. The analysis of the NBI, chlorophylls and polyphenols showed an increase in the nitrogen status of plants treated with ABP-B9 (Fig. 14A), in particular the significant increase in chlorophylls (Fig. 14B) and anthocyanins (Fig. 14C) can result in increased photosynthetic capacity.

### 3.2. Evaluation of the effect of ABP-B9 on the development and physiological state of Loire melon plants

To evaluate the biostimulant effect of ABP-B9 in commercial melon cultivation, a trial was carried out using the Loire variety (cantaloup type), in a plot located in San Pedro del Pinatar (Murcia). Two control treatments were carried out (inoculated with 5 ml of water) and ABP-B9 (5 ml at 2.1 x 10⁸ CFU/ml), 18 plants/treatment. The seeds were germinated and planted in a seedbed, and 5 days before transplanting, 5 ml of each inoculum was applied to the crop plot. Three replicates/treatment were made (6 plants/replica) that were distributed in randomised blocks, separated from each other with untreated plants to avoid contamination. A visual monitoring of the development of the crop was carried out, and the incidence of diseases was evaluated. Once the test was completed (61 dpt), the weight of the aerial part, plant length and dry weight of the root system were determined.

The analyses showed that the application of ABP-B9 did not produce a negative effect on the development of the treated plants and did produce an increase in the length of the plants and the dry weight of the root system (Fig. 15). Furthermore, the production of each of the plants was analysed, which allowed the verification that, although the application of ABP-B9 did not induce an increase in the number of melons produced, an increase in their weight, its diameter and thickness of the pulp was observed (Fig. 16). The quantification of the ABP-B9 population in the root system of plants at the end of the crop cycle showed that the population was stabilised at the usual levels detected of this microorganism in the root system of untreated plants (Fig. 15C).

### 4. EVALUATION OF THE BIOSTIMULANT CAPACITY IN CHERRY TOMATO

To evaluate the effect of ABP-B9 on the development of the tomato crop, a trialwas carried out in pots (1L) in a greenhouse with substrate *"Floragard Grow Mix",* using a cherry tomato variety (BayPlant seedbed). Three treatments were tested: Control (water), ABP-B9 (10⁸ CFU/ml) and ABP-B9(1/10) (10⁷ CFU/ml), 36 plants/treatment, distributed in random blocks (4 or 5 plants/block). Three applications of the treatments were carried out, one at the beginning of the assay, at 15 and at 30 days. During cultivation, the visual monitoring of the development of the plants was exhaustive, 3 tomato collections were made, at 53, 60 and 70 days, the last one coinciding with the end of the trial, at which time all the plants were sampled.

The number of clusters, the flowers and tomatoes produced were counted; each plant was weightedand the diameter of the stem measured. Samples of full leaves were taken from the upper third of plants from each treatment and stomatal conductance was determined (C.e. µmol m²s⁻¹) with a CS-1 diffusion porometer (Meter Group). The acidity and brix degrees of the tomatoes from the first 3 harvests were determined by refractometry and electroconductivity (ATAGO Brix-Acidity Meters). The ABP-B9 population in the root system of 6 plants was quantified by real-time quantitative PCR using SYBR Green.

The analysis of the number of tomatoes produced in the different harvests showed that the application of ABP-B9 led to an increase in the average weight of the tomatoes, and in the total number of these (mature and immature), this increase being slightly higher with the ABP-B9 treatment (1/10) (Fig. 17A) than with ABP-B9 (Table 2 and Fig 17A). The extrapolation of the data to the production of 100 plants maintained the trend, with the greater increase with ABP-B9 (1/10) followed by ABP-B9 (Fig. 17D).

**Table 2. Data obtained after harvesting tomatoes from the plants of the different treatments.**

| | **Average no. of tomatoes per plant** | **Standard error** | **No. of plants** | **Total tomatoes per tto** | **Tomatoes in 100 plants** |
|---|---|---|---|---|---|
| **Control** | 135.90 | 4.04 | 23 | 3,126 | 13,591.30 |
| **ABP-B9** | 143.30 | 4.85 | 21 | 3,010 | 14,333.30 |
| **ABP-B9 (1/10)** | 147.30 | 4.46 | 20 | 2,946 | 14,730.00 |

The analysis of stomatal conductance (5 plants/treatment) did not show significant differences between the control treatment and the treatments inoculated with ABP-B9 (Fig. 18A), indicating that ABP-B9 did not produce biotic stress to the plants. This fact could indicate that plants treated with ABP-B9 are more photosynthetically efficient than control plants, since with the same stomatal conductance they are more productive both in number and in weight of tomatoes (Fig. 17B and C). The determination of the acidity and sugar content parameters showed that the application of ABP-B9 seems to decrease the acidity and sugar content (Fig. 18B and C). This application did not produce significant variation in the number of clusters, of flowers or plant weight, but a significant increase in flower production was observed in the ABP-B9 treatment (1/10) compared to the other treatments (Fig. 17E). The quantification of the ABP-B9 population present in the root system of the plants confirmed its presence in the ABP-B9 and ABP-B9(1/10) treatments. being higher in the ABP-B9 treatment (Fig. 18D).

### 5. EVALUATION OF THE BIOSTIMULANT CAPACITY IN SPINACH

The biostimulant effect of ABP-B9 in spinach was analysed using the Elgiga variety, and 2 treatments were carried out, control and ABP-B9, 2,100 plants/treatment with 3 replicates of 700 plants, in blocks of 75 x 140 cm, separated with untreated plants to avoid cross contamination. Spinach seeds were sown directly at a density of 7.5 million plants/ha, 20 days after sowing, when the plants had developed the first true leaf, the treatments were applied with the help of a 10L spray backpack, 10.5 L (3.5 L/ replica), with constant outlet pressure, each spinach line was watered by sprinkler 4 times. The ABP-B9 treatment was applied at a concentration of 5 x 10⁸ CFU/ml. Exhaustive visual monitoring of crop development and disease incidence was conducted.

The trial ended 35 days after application of the treatments, all the plants were sampled. 120 plants/treatment (40 plants/replica) were taken whole and the aerial part length measured and weighted both the aerial part and the root system; the dry weight of their roots was determined after washing followed by drying at 70°C for 5 days. The analysis of the effect on crop yield was completed by determining the number and weight of the leaves of the remaining plants.

The results showed that the application of ABP-B9 produced a significant increase in the length and weight of the aerial part (Fig. 19 A and B) compared to the control plants, as well as a significant increase in root dry weight (Fig. 20A) and the average weight of the plants (4% compared to the control), (Fig. 19C). When the yield was estimated at 100 m² of the crop for each treatment, it was observed that the increase produced by the ABP-B9 treatment was 7% compared to the control (Fig. 19D). The quantification of the ABP-B9 population in the root system 35 days after inoculation showed a population 2 orders of magnitude higher in the treatment with the bacteria than in the control (Fig. 20B) and plants treated with ABP-B9 had greater root length (Fig. 20C).

### 6. EVALUATION OF THE BIOSTIMULANT CAPACITY IN CELERY

The evaluation of the biostimulant and biocontrol effect of ABP-B9 in celery crops was carried out using the PH 535 variety, intended for IV range products, therefore the absence of damage to leaves, the length and weight of the stems are valued; 2 treatments were carried out (control and ABP-B9), 100 plants/treatment, (3 replicas of 33 plants), were distributed in randomised blocks, separated with untreated plants to avoid cross contamination. The trial was carried out on a commercial cultivation farm located in Águilas (Murcia) with a planting density of 94,444 plants/ha. The seeds were planted in a seedbed and 5 days before transplanting to the field the seedlings were inoculated with 5 ml/plant of water (control) or ABP-B9 (10⁸ CFU/ml).

Exhaustive visual monitoring of crop development and disease incidence was conducted. Five days after the application of the treatments, greater development was observed in the plants treated with ABP-B9 than the control plants (Fig. 21). During cultivation, no incidence of diseases was observed, and the plants developed normally. After about 3 months and 20 days (112 days) from transplant, the trial was carried out, the length of the plants was measured, the aerial part was weighted and the dry weight of the roots were determined. The results of these analyses showed that plants treated with ABP-B9 showed greater development than the control, with a greater length (Fig. 22A), greater weight of the aerial part (Fig. 22B), as well as an increase in the dry weight of the root system (Fig. 22C).

## Claims

1. A strain of *Pseudomonas seleniipraecipitans* with deposit number CECT 30310.

2. A composition comprising the strain according to claim 1.

3. The composition according to claim 2 which further comprises at least one excipient and/or a bacterial culture medium.

4. Use of the strain according to claim 1 or the composition according to claim 2 or 3 as a biostimulant for the growth of a plant, as an agent inducing stress resistance in a plant and/or as a biocontrol agent against a plant pathogen.

5. Use according to claim 4, wherein the plant belongs to the genus *Lactuca* spp, *Cucumis* spp, *Spinacea* spp., *Solanum* spp., or *Apium* spp.

6. Use according to claim 4 or 5, wherein the plant belongs to the species *Lactuca sativa* sp., *Cucumis melo* sp., *Spinacea oleracea* sp., *Solanum lycopersicum* sp., or *Apium graveolens* sp.

7. Use according to any one of claims 4 to 6, wherein the pathogen is a fungus or a virus.

8. Use according to claim 7, wherein, the fungus belongs to the genus *Olpidium* spp.

9. Use according to claim 8, wherein the fungus belongs to the species *Olpidium virulentus* sp.

10. Use according to claim 7, wherein the virus belongs to the genus *Ophiovirus* spp. or *Varicosavirus* spp.

11. Use according to claim 10, wherein the virus belonging to the genus *Ophiovirus* spp. is the Mirafiori lettuce big-vein virus (MiLBVV) and/or the virus that belongs to the genus *Varicosavirus* spp. is the lettuce big-vein associated virus (LBVaV).

12. Method for increasing or promoting the growth of a plant, to increase the stress resistance of a plant and/or for the biocontrol of pathogens in a plant, that comprises:
(i) administering the strain of *Pseudomonas seleniipraecipitans* according to claim 1 or the composition according to claims 2 or 3 to a plant or plant material isolated from said plant.

13. The method according to claim 12, wherein the contact in step (i) is carried out by applying the strain or the composition in liquid form, in solid form or hydroponically, preferably in liquid form.

14. The method according to claim 12 or 13, wherein the strain or composition of the invention is administered at least once, preferably between one and five times, more preferably between one and three times.

15. The method according to any one of claims 12 to 14, wherein the plant belongs to the genus *Lactuca* spp., *Cucumis* spp., *Spinacea* spp., *Solanum* spp., *or Apium* spp.

16. The method according to any one of claims 12 to 15, wherein the plant belongs to the species *Lactuca sativa* sp., *Cucumis melo* sp., *Spinacea oleracea* sp., *Solanum lycopersicum* sp., or *Apium graveolens* sp.

17. The method according to any one of claims 12 to 16, wherein the pathogen is a fungus or a virus.

18. The method according to claim 17, wherein the fungus belongs to the genus *Olpidium* spp., preferably to the species *Olpidium virulentus* sp.

19. The method according to claim 17, wherein the virus belongs to the genus *Ophiovirus* spp. or *Varicosavirus* spp.

20. The method according to claim 19, wherein the virus belonging to the genus Ophiovirus spp. is the Mirafiori lettuce big-vein virus (MiLBVV) and/or the virus that belongs to the genus *Varicosavirus* spp. is the lettuce big-vein associated virus (LBVaV).
